# EUROPEAN PATENT APPLICATION

(11) **EP 3 556 343 A1**
(43) Date of publication of application: **23.10.2019**
(21) Application number: 17881725.0
(22) Date of filing: 14.12.2017
(51) Int. Cl.: A61K 8/34, A61K 8/06, A61K 8/36, A61K 8/39, A61K 8/46

(54) **OIL-IN-WATER TYPE COMPOSITION**

(30) Priority: 16.12.2016 JP 2016244356; 15.09.2017 JP 2017178222
(71) Applicant: Shiseido Company, Ltd., Tokyo 104-0061 (JP)
(72) Inventor: UCHIYAMA Tomoya, Yokohama-shi Kanagawa 224-8558 (JP); DAIDOGUCHI Noriko, Yokohama-shi Kanagawa 224-8558 (JP); SUZUKI Kazuaki, Yokohama-shi Kanagawa 224-8558 (JP); YABUSAKI Yusuke, Yokohama-shi Kanagawa 224-8558 (JP)
(74) Representative: Ter Meer Steinmeister & Partner
(86) International application number: PCT/JP2017/044957
(87) International publication number: WO 2018/110659

(57) **Abstract**

An oil-in-water emulsion composition includes component (A): from 0.1 to 5% by mass of a hydrophilic nonionic surfactant, component (B): more than 0.02% by mass to less than 0.5% by mass of an anionic surfactant, component (C): from 0.5 to 6% by mass of a higher alcohol, and component (D): from 2 to 30 mmol/100 g of an organic acid and/or a salt and/or derivative thereof. The component (A) includes a polyoxyalkylene alkyl ether and/or a polyalkylene glycol fatty acid ester.

## Description

### Cross Reference to Related Applications

The present invention is based upon and claims the benefit of the priority of Japanese Patent Applications No. 2016-244356 (filed on December 16, 2016) and No. 2017-178222 (filed on September 15, 2017), the disclosure of which is incorporated herein in its entirety by reference.

### Technical Field

The present disclosure relates to an oil-in-water emulsion composition. For example, the present disclosure relates to an oil-in-water emulsion composition applicable to cosmetics and external preparations for skin.

### Background Art

Patent Literature 1 discloses an oil-in-water emulsion cosmetic containing: 0.2 to 5.0% by mass of a sorbitan polyoxyethylene fatty acid ester wherein the fatty acid moiety is derived from a C₁₄₋₁₈ linear saturated fatty acid; at least one type selected from anionic surfactants, which are selected from N-acyl methyl taurates, acyl glutamates, acyl lactatess and fatty acid soaps, and nonionic surfactants having an I.O.B value of 1.6 or greater; 0.2 to 6.0% by mass of at least one type selected from C₁₄₋₂₂ non-branched higher alcohols; 3.0% by mass or less of at least one type selected from glycerin monoalkyl esters, glycerin monoalkyl ethers, sorbitan monoalkyl esters, sorbitan monoalkyl esters, sterols, and branched higher alcohols; and 0.1 to 3.0% by mass of an organic acid or a salt thereof.

Patent Literature 2 describes a method for producing an O/W emulsion composition having very small emulsion particle diameters, the O/W emulsion composition employing an anionic surfactant and a higher aliphatic alcohol that form an α-gel in water. Patent Literature 2 discloses the O/W emulsion composition containing 0.02% by mass of citric acid and 0.08% by mass of sodium citrate.

### Citation List

### Patent Literature

Patent Literature 1: Japanese Unexamined Patent Publication No. 2008-88129A
Patent Literature 2: International Publication WO2013/061712A1

### Summary of Invention

### Technical Problem

Each content of the above Patent Literatures is incorporated herein in its entirety by reference. The following analysis is provided from the perspective of the present disclosure.

The oil-in-water emulsion cosmetic of Patent Literature 1 aims at obtaining a fresh feel upon use. Thus, with the oil-in-water emulsion cosmetic of Patent Literature 1, it is difficult to achieve feelings other than a fresh feel upon use, for example, feelings such as "skin smoothness (soft feeling)", "moistness", and "swift finish" upon use.

Further, the addition of an active ingredient potentially of improving skin such as organic acids (including salts and derivatives thereof) to an oil-in-water composition containing a higher alcohol and a nonionic surfactant, as disclosed in Patent Literature 1, causes the emulsion particles to unite, thereby making it difficult to obtain a stable emulsion.

When the amount of the active ingredient added is small, as disclosed in Patent Literature 2, emulsion stability is hardly affected by the active ingredient. However, increasing the amount of the active ingredient added in order to enhance the effects brought about by the active ingredient greatly affects emulsion stability.

Thus, there is a demand for an oil-in-water composition that achieves the aforementioned feelings upon use and that also has excellent emulsion stability even when active ingredients are included as described above.

### Solution to Problem

According to a first aspect of the present disclosure, an oil-in-water emulsion composition is provided, the composition comprising: component (A): from 0.1 to 5% by mass of a hydrophilic nonionic surfactant; component (B): more than 0.02% by mass to less than 0.5% by mass of an anionic surfactant; component (C): from 0.5 to 6% by mass of a higher alcohol; and component (D): from 2 to 30 mmol/100 g of an organic acid and/or a salt and/or derivative thereof. The component (A) includes a polyoxyalkylene alkyl ether and/or a polyalkylene glycol fatty acid ester.

### Advantageous Effects of Invention

With the composition of the present disclosure, it is possible to provide, upon use, a user with such feelings as skin smoothness (soft feeling), moistness, and swift finish. Further, the composition of the present disclosure has excellent emulsion stability even when an active ingredient potentially of improving skin, such as organic acids, is included.

### Description of Embodiments

Preferred modes of the above aspect will be described below.

According to a preferred mode of the first aspect, the component (A) has an HLB of 9 or greater.

According to a preferred mode of the first aspect, the component (B) includes at least one compound selected from the group consisting of N-acyl methyl taurates, N-acyl glutamates, and phosphate ester salts.

According to a preferred mode of the first aspect, the component (C) includes a C₁₄₋₂₂ linear saturated alkyl group.

According to a preferred mode of the first aspect, the component (D) includes citric acid, 4-methoxysalicylic acid, L-ascorbic acid, kojic acid, and/or 1-piperidinepropionic acid, and/or a salt and/or derivative thereof.

According to a preferred mode of the first aspect, the composition further comprises component (E): from 0.2 to 1% by mass of a thickener.

According to a preferred mode of the first aspect, the component (E) includes a homopolymer and/or copolymer of 2-acrylamido-2-methylsulfonic acid or a salt thereof.

According to a preferred mode of the first aspect, the composition further comprises component (F): from 0.1 to 2% by mass of a lipophilic nonionic surfactant.

According to a preferred mode of the first aspect, the lipophilic nonionic surfactant has an HLB of from 2 to 5.

According to a preferred mode of the first aspect, the component (F) includes a sorbitan fatty acid ester and/or a glycerin fatty acid ester.

According to a preferred mode of the first aspect, a value found by dividing the mass of the component (B) by the total mass of the component (A) and the component (F) is from 0.003 to 0.3.

According to a preferred mode of the first aspect, the component (A) includes a polyoxyalkylene alkyl ether. The average number of moles of polyoxyalkylene groups added in the polyoxyalkylene alkyl ether is from 10 to 40.

According to a preferred mode of the first aspect, the component (A) includes a polyalkylene glycol fatty acid ester. The average number of moles of polyalkylene glycol groups added in the polyalkylene glycol fatty acid ester is from 25 to 55.

According to a preferred mode of the first aspect, an average particle size of emulsion particles is less than 5 µm.

According to a preferred mode of the first aspect, the composition has a hardness of from 8 to 22.

A composition according to a first embodiment of the present disclosure is described below. The composition of the present disclosure is an oil-in-water emulsion composition. The composition of the present disclosure is applicable, for example, to cosmetics and external preparations for skin.

In the following description, POE is an abbreviation of polyoxyethylene, POP is an abbreviation of polyoxypropylene, and the number in parentheses after POE or POP indicates the average number of moles of POE groups or POP groups added in the compound in question.

PEG is an abbreviation of polyethylene glycol, and the number in parentheses after PEG indicates the average number of moles of PEG groups added in the compound in question.

The composition of the present disclosure contains: (A) a hydrophilic nonionic surfactant; (B) an anionic surfactant; (C) a higher alcohol; and (D) an organic acid and/or a salt and/or derivative thereof.

### [(A) Hydrophilic Nonionic Surfactant]

The hydrophilic nonionic surfactant includes a polyoxyalkylene alkyl ether and/or a polyalkylene glycol fatty acid ester. The hydrophile-lipophile balance (HLB) of the component (A) is preferably 9 or greater. By using such a nonionic surfactant, it is possible to provide, upon use, a user with such feelings as skin smoothness (soft feeling), moistness, and swift finish.

In the present disclosure, "skin smoothness (soft feeling)" refers to a moist feeling caused by a moisturizer and a feeling that moisture is being retained in the skin, which is caused by the effect of occlusion by a coating film. "Swift finish" refers to a feeling that the composition breaks up quickly (the gel-sol transition point is reached quickly) and immediately conforms to the skin, without lingering sliminess on the skin.

Examples of the polyoxyalkylene alkyl ether may include polyoxyethylene behenyl ether and polyoxyethylene stearyl ether. The average number of moles of polyoxyalkylene groups added is preferably 10 or greater, more preferably 20 or greater. The average number of moles of polyoxyalkylene groups added is preferably 40 or less, more preferably 30 or less. Emulsion stability can be improved when the average number of moles of polyoxyalkylene groups added is within the aforementioned range. Examples of commercially available products of polyoxyethylene alkyl ethers may include NIKKOL® BB-20, BB-30, etc. (from Nikko Chemicals Co., Ltd.).

Examples of the polyalkylene glycol fatty acid ester may include polyethylene glycol monostearate and polyethylene glycol monooleate. The average number of moles of polyoxyalkylene groups added is preferably 25 or greater. The average number of moles of polyoxyalkylene groups added is preferably 55 or less, more preferably 40 or less. Emulsion stability can be improved when the average number of moles of polyoxyalkylene groups added is within the aforementioned range. Examples of commercially available products of polyethylene glycol fatty acid esters may include NIKKOL® MYS-40V, MYS-55V, etc. (from Nikko Chemicals Co., Ltd.).

The composition of the present disclosure may contain other hydrophilic nonionic surfactant having an HLB of 9 or greater, in amounts that do not inhibit the effects of the composition of the present disclosure. Examples of other hydrophilic nonionic surfactants may include POE alkyl ethers, such as POE (2) lauryl ether, POE (4.2) lauryl ether, POE (9) lauryl ether, POE (5.5) cetyl ether, POE (7) cetyl ether, POE (10) cetyl ether, POE (15) cetyl ether, POE (20) cetyl ether, POE (23) cetyl ether, POE (4) stearyl ether, POE (20) stearyl ether, POE (7) oleyl ether, POE (10) oleyl ether, POE (15) oleyl ether, POE (20) oleyl ether, POE (50) oleyl ether, POE (10) behenyl ether, POE (20) behenyl ether, POE (30) behenyl ether, POE (2) (C₁₂₋₁₅) alkyl ether, POE (4) (C₁₂₋₁₅) alkyl ether, POE (10) (C₁₂₋₁₅) alkyl ether, POE (5) secondary alkyl ether, POE (7) secondary alkyl ether, POE (9) alkyl ether, and POE (12) alkyl ether.

Examples of other hydrophilic nonionic surfactants may include POE/POP alkyl ethers, such as POE (1) polyoxypropylene (POP) (4) cetyl ether, POE (10) POP (4) cetyl ether, POE (20) POP (8) cetyl ether, POE (20) POP (6) decyltetradecyl ether, and POE (30) POP (6) decyltetradecyl ether.

Examples of other hydrophilic nonionic surfactants may include polyethylene glycol (abbreviated hereinafter as PEG) fatty acid esters, such as PEG (10) monolaurate, PEG (10) monostearate, PEG (25) monostearate, PEG (40) monostearate, PEG (45) monostearate, PEG (55) monostearate, PEG (100) monostearate, PEG (10) monooleate, PEG distearate, and PEG diisostearate. Examples of other hydrophilic nonionic surfactants may include polyglycerin fatty acid esters, such as hexaglyceryl monolaurate, hexaglyceryl monomyristate, hexaglyceryl monostearate, hexaglyceryl monooleate, decaglyceryl monolaurate, decaglyceryl monomyristate, decaglyceryl monostearate, decaglyceryl monoisostearate, decaglyceryl monooleate, decaglyceryl distearate, and decaglyceryl diisostearate.

Examples of other hydrophilic nonionic surfactants may include polyoxyethylene (POE) glycerin fatty acid esters, such as POE (5) glyceryl monostearate, POE (15) glyceryl monostearate, POE (5) glyceryl monooleate, and POE (15) glyceryl monooleate.

Examples of other hydrophilic nonionic surfactants may include POE glyceryl isostearates, such as PEG (8) glyceryl isostearate, PEG (10) glyceryl isostearate, PEG (15) glyceryl isostearate, PEG (20) glyceryl isostearate, PEG (25) glyceryl isostearate, PEG (30) glyceryl isostearate, PEG (40) glyceryl isostearate, PEG (50) glyceryl isostearate, and PEG (60) glyceryl isostearate.

The content of the component (A) to the mass of the composition is preferably 0.1% by mass or greater, more preferably 0.4% by mass or greater. If the component (A) is less than 0.1% by mass, oily components cannot be emulsified stably. The content of the component (A) to the mass of the composition is preferably 5% by mass or less, more preferably 2% by mass or less, even more preferably 1% by mass or less. If the component (A) exceeds 5% by mass, the feel upon use will deteriorate and become sticky.

### [(B) Anionic Surfactant]

Examples of the anionic surfactants that may be used may include fatty acid soap (such as sodium laurate, and sodium palmitate); higher alkyl sulfate ester salt (such as sodium lauryl sulfate, and potassium lauryl sulfate); alkyl ether sulfate ester salt (such as POE-lauryl sulfate triethanolamine, and sodium POE-lauryl sulfate); N-acyl sarcosinic acid (such as sodium lauroyl sarcocinate); higher fatty acid amide sulfonate (such as sodium N-stearoyl-N-methyltaurate, sodium N-myristoyl-N-methyltaurate, sodium methyl cocoyl taurate, and sodium laurylmethyl taurate); phosphate ester salt (sodium POE-oleylether phosphate, POE-stearylether phosphate, potassium cetyl phosphate); sulfosuccinate (such as sodium di-2-ethylhexyl sulfosuccinate, sodium monolauroyl monoethanolamide polyethylene sulfosuccinate, and sodium lauryl polypropylene glycol sulfosuccinate); alkylbenzene sulfonate (such as sodium linear dodecylbenzene sulfonate, triethanolamine linear dodeylbenzene sulfonate, and linear dodecylbenzene sulfonate); higher fatty acid ester sulfate ester salt (such as sodium hydrogenated gryceryl cocoate sulfate); N-acyl glutamate (such as monosodium N-lauroyl glutamate, disodium N-stearoyl glutamate, and monosodium N-myristoyl-L-glutamate); sulfonated oil (such as Turkey red oil); POE-alkyl ether carboxylic acid; POE-alkyl aryl ether carboxylate; α-olefine sulfonate; higher fatty acid ester sulfonate; secondary alcohol sulfate ester salt; higher fatty acid alkylolamide sulfate ester salt; sodium lauroyl monoethanolamide succinate; N-palmitoyl asparaginate ditriethanolamine; sodium casein; and the like.

Among the above, it is preferred that the anionic surfactant includes, for example, at least one compound selected from the group consisting of N-acyl methyl taurates, N-acyl glutamates, and phosphate ester salts. Examples of anionic surfactants that may suitably be used include stearoyl methyl taurates, lauroyl methyl taurates, myristoyl methyl taurates, coconut oil fatty acid methyl taurates, N-myristoyl-L-glutamates, N-stearoyl-L-glutamates, and cetyl phosphates. Adding the anionic surfactant can achieve a stable emulsion state, even when the later-described component (D), such as an organic acid, is added.

The content of the component (B) to the mass of the composition is preferably greater than 0.02% by mass, more preferably 0.03% by mass or greater, even more preferably 0.04% by mass or greater. If the component (B) is 0.02% by mass or less, a stable emulsion state cannot be achieved when the content by percentage of the component (D) is high. The content of the component (B) to the mass of the composition is preferably less than 0.5% by mass, more preferably 0.4% by mass or less, more preferably 0.3% by mass or less, even more preferably 0.2% by mass or less, even more preferably 0.1% by mass or less. If the component (B) is 0.5% by mass or greater, sliminess will linger at the time of application to the skin.

### [(C) Higher Alcohol]

Examples of the higher alcohol that may be used may include linear alcohol (such as lauryl alcohol, cetyl alcohol, stearyl alcohol, behenyl alcohol, myristyl alcohol, oleyl alcohol, and cetostearyl alcohol); branched-chain alcohol (such as monostearylglycerin ether (batyl alcohol), 2-decyltetradecinol, lanolin alcohol, cholesterol, phytosterol, hexyldodecanol, isostearyl alcohol, and octyldodecanol) and the like.

In the composition of the present disclosure, it is particularly preferred to include a C₁₄₋₂₂ higher alcohol including a linear saturated alkyl group. For example, stearyl alcohol and/or behenyl alcohol can suitably be used. The feel upon use can be improved by using such a higher alcohol.

The content of the component (C) to the mass of the composition is preferably 0.5% by mass or greater, more preferably 1% by mass or greater, even more preferably 2% by mass or greater. If the component (C) is less than 0.5% by mass, the feel upon use cannot be improved. The content of the component (C) to the mass of the composition is preferably 6% by mass or less, more preferably 5% by mass or less, even more preferably 4% by mass or less. If the component (C) exceeds 6% by mass, hardness will increase and the feel upon use will deteriorate.

### (D) Organic Acid and/or Salt and/or Derivative Thereof:

The component (D) imparts, to the composition, the effect of improving skin quality when applied to the skin. Herein, an organic acid is an organic compound that includes a carboxyl group and that ionizes to produce a hydrogen ion. This compound encompasses amino acids and analogs thereof. Examples of the component (D) may include at least one of citric acid, 4-methoxysalicylic acid, L-ascorbic acid, kojic acid, carnosine, tranexamic acid and 1-piperidinepropionic acid, and salts and derivatives thereof.

The content of the component (D) to 100 g of the composition is preferably 2×10⁻³ mol or greater, more preferably 4×10⁻³ mol or greater, even more preferably 6×10⁻³ mol or greater. If the component (D) is less than 2×10⁻³ mol, the effects based on the component (D) will deteriorate. The content of the component (D) to 100 g of the composition is preferably 3×10⁻² mol or less, more preferably 2.5×10⁻² mol or less, even more preferably 2×10⁻² mol or less. If the component (D) exceeds 3×10⁻² mol, emulsion stability will deteriorate.

### (E) Thickener:

In addition to the aforementioned components, the composition of the present disclosure may further contain (E) a thickener.

Examples of the thickener may include taurate-based synthetic polymers and/or acrylate-based synthetic polymers.

For the taurate-based polymeric thickener, it is possible to use, for example, polymers and/or copolymers (including crosslinked polymers) including 2-acrylamido-2-propanesulfonic acid (acryloyldimethyl taurine acid) or a salt thereof (AMPS structure) as a constitutional unit. For such thickeners, it is possible to use, for example, at least one selected from an ammonium acryloyldimethyltaurate/beheneth-25 methacrylate crosspolymer (Aristoflex® HMB from Clariant (Japan) K.K.), ammonium acryloyldimethyltaurate/vinylpyrrolidone copolymer (Aristoflex® AVC from Clariant (Japan) K.K.), ammonium acryloyldimethyltaurate/carboxyethyl acrylate crosspolymer (Aristoflex® TAC from Clariant (Japan) K.K.), polyacrylate crosspolymer-11 (Aristoflex® Velvet from Clariant (Japan) K.K.), dimethylacrylamide/sodium acryloyldimethyltaurate crosspolymer, hydroxyethyl acrylate/sodium acryloyldimethyltaurate copolymer (SEPINOV EMT10 PINOV from Seppic), sodium acrylate/acryloyldimethyl taurine/dimethylacrylamide crosspolymer (SEPINOV P88 from Seppic), sodium acrylate/sodium acryloyldimethyltaurate copolymer (SIMULGEL EG from Seppic), sodium acryloyldimethyltaurate/methacrylamidolauric acid copolymer (AMO-51 from Daitoh Chemical Co., Ltd.), and acrylamide/sodium acryloyldimethyltaurate/acrylic acid copolymer (Acudyne SCP from Dow Chemical Company).

For the acrylate-based synthetic polymeric thickener, it is possible to use, for example, acrylate/steareth-20 methacrylate copolymer (Aculyn® 22 from Dow Chemical Company) and acrylate/C₁₀₋₃₀ alkyl acrylate crosspolymer (PEMULEN® TR-2).

Examples of other thickeners may include gum arabic, carrageenan, karaya gum, tragacanth gum, carob gum, quince seed (marmelo), casein, dextrin, gelatin, sodium pectate, sodium alginate, methyl cellulose, ethyl cellulose, carboxymethyl cellulose (CMC), hydroxyethyl cellulose, hydroxypropyl cellulose, polyvinyl alcohol (PVA), polyvinylmethyl ether (PVM), PVP (polyvinyl pyrrolidone), polysodium acrylate, carboxyvinyl polymer, locust bean gum, guar gum, tamarind gum, dialkyldimethylammonium sulfate cellulose, xanthan gum, aluminum magnesium silicate, bentonite, hectorite, aluminum magnesium silicate (Veegum), sodium magnesium silicate (Laponite), and silicic acid anhydride.

In the composition of the present disclosure, the content by percentage (total amount) of the thickener(s) to the mass of the composition is preferably 0.2% by mass or greater, more preferably 0.3% by mass or greater. If the content by percentage of the thickener is less than 0.2% by mass, emulsion stability and the feel upon use will deteriorate. The content by percentage of the thickener to the mass of the composition is preferably 1% by mass or less, more preferably 0.6% by mass or less. If the content by percentage of the thickener exceeds 1% by mass, the feel upon use will deteriorate.

### [(F) Lipophilic Nonionic Surfactant]

The composition of the present disclosure may further contain (F) a lipophilic nonionic surfactant. The HLB of the component (F) is preferably from 2 to 5.

Examples of lipophilic nonionic surfactants having an HLB of from 2 to 5 may include POE (2) stearyl ether, self-emulsifying propylene glycol monostearate, glyceryl myristate, glyceryl monostearate, self-emulsifying glyceryl monostearate, glyceryl monoisostearate, glyceryl monooleate, hexaglyceryl tristearate, decaglyceryl pentastearate, decaglyceryl pentaisostearate, decaglyceryl pentaoleate, sorbitan monostearate, sorbitan tristearate, sorbitan monoisostearate, sorbitan sesquiisostearate, sorbitan monooleate, POE (6) sorbitol hexastearate, POE (3) castor oil, PEG (2) monostearate, ethylene glycol monostearate, and PEG (2) stearate.

Among the above, in the composition of the present disclosure, the component (F) is preferably, for example, a sorbitan fatty acid ester and/or a glycerin fatty acid ester. For the component (F), it is possible to use, for example, sorbitan tristearate, sorbitan monostearate, and self-emulsifying glyceryl monostearate.

The content of the component (F) to the mass of the composition is preferably 0.1% by mass or greater. If the component (F) is less than 0.1% by mass, emulsion stability will deteriorate. The content of the component (F) to the mass of the composition is preferably 2% by mass or less.

### [Mass Ratio of Component (B) to Component (A) and Component (F)]

In the composition of the present disclosure, it is considered that the feel upon use is improved by the nonionic surfactants, i.e., the components (A) and (F). Also, it is considered that emulsification is stabilized by the anionic surfactant, i.e., the component (B). In order to improve both the feel upon use and emulsion stability, the mass ratio between the nonionic surfactants and anionic surfactants, i.e., the mass of the component (B) to the total mass of the components (A) and (F), is preferably 0.003 or greater, more preferably 0.02 or greater. Further, the mass of the component (B) to the total mass of the components (A) and (F) is preferably 0.3 or less, more preferably 0.2 or less, even more preferably 0.1 or less.

### [(G) Others]

The oil-in-water emulsion composition of the present disclosure may include, as appropriate and as necessary, other components-such as aqueous solvents, amphoteric surfactants, cationic surfactants, powder bodies, moisturizers, water-soluble polymers, oily components, film-forming agents, UV absorbers, metal ion sequestering agents, amino acids, organic amines, polymer emulsions, silicone elastomers, pH adjusters, skin nutrients, vitamins, antioxidants, antioxidant aids, and perfumes-in amounts that do not inhibit the effects of the present disclosure.

Examples of aqueous solvents may include water, alcohols, moisturizers, and mixtures thereof.

With respect to water, water used for such as cosmetics and quasi-pharmaceutical products can be used, including e.g., purified water, ion-exchanged water, and tap water. Depending on the purpose, the aqueous phase may further include a water-soluble alcohol.

Examples of water-soluble alcohols may include at least one type selected from lower alcohols, polyhydric alcohols, polyhydric alcohol polymers, dihydric alcohol alkyl ethers, dihydric alcohol alkyl ethers, dihydric alcohol ether esters, glycerin monoalkyl ethers, sugar alcohols, monosaccharides, oligosaccharides, polysaccharides, and derivatives of the above.

Examples of the lower alcohol may include ethanol, propanol, isopropanol, isobutyl alcohol, t-butyl alcohol, and the like.

Examples of the polyhydric alcohol may include dihydric alcohol (such as ethylene glycol, propylen glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol, etc); trihydric alcohol (such as glycerin, trimethylolpropane, etc); tetrahydric alcohol (such as such as pentaerythritol such as 1,2,6-hexanetriol, etc); pentahydric alcohol (such as xylitol, etc); hexahydric alcohol (such as sorbitol, mannitol, etc); polyhydric alcohol polymer (such as diethylene glycol, dipropylene glycol, triethylene glycol, polypropylene glycol, tetraethylene glycol, diglycerin, polyethylene glycol, triglycerin, tetraglycerin, polyglycerin, etc); dihydric alcohol alkyl ethers (such as ethylene glycol monomethyl ether, ethylene glycol monoethyl ether, ethylene glycol monobutyl ether, ethylene glycol monomphenyl ether, ethylene glycol monohexyl ether, ethylene glycol mono2-methylhexyl ether, ethylene glycol isoamyl ether, ethylene glycol benzil ether, ethylene glycol isopropyl ether, ethylene glycol dimethyl ether, ethylene glycol diethyl ether, ethylene glycol dibutyl ether, etc); dihydric alcohol alkyl ethers (such as diethylene glycol monomethyl ether, diethylene glycol monoethyl ether, diethylene glycol monombutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether, etc); dihydric alcohol ether ethers (such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disaccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate, etc); glycerin monoalkyl ether (such as chimyl alcohol, selachyl alcohol, batyl alcohol, etc); sugar alcohol (such as sorbitol, maltitol, maltotriose, mannitol, sucrose, erythritol, glucose, fructose, starch sugar, maltose, xylitose, starch sugar hydrogenated alcohol, etc); glycolide, tetrahydrofurfuryl alcohol; POE-tetrahydrofurfuryl alcohol; POP/POE-butyl ether; tripolyoxypropylene glycerin ether; POP-glycerin ether; POP-glycerin ether phosphoric acid; POP/POE-pentaerythritol ether; polyglycerin, and the like.

Examples of the monosaccharides may include at least one selected from triose (such as D-glyceryl aldehyde, dihydroxyacetone, etc); tetrose (such as D-erythrose, D-erythrulose, D-threose, erythritol, etc); pentaose (such as L-arabinose, D-xylose, L-lyxose, D-arabinose, D-ribose, D-ribulose, D-xylulose, L-xylulose, etc); hexalose (such as D-glucose, D-talose, D-psicose, D-galactose, D-fructose, L-galactose, L-mannose, D-tagatose, etc); heptose (such as aldoheptose, heplose, etc); octose (such as octulose, etc); deoxy sugar (such as 2-deoxy-D-ribose, 6-deoxy-L-galactose, 6-deoxy-L-mannose, etc); amino sugar (such as D-glucosamine, D-galactosamine, sialic acid, amino uronic acid, muramic acid, etc); uronic acid (such as D-grucuronic acid, D-mannuronic acid, L-guluronic acid, D-garacturonic acid, L-iduronic acid, etc) and the like.

Examples of the oligosaccharide may include at least one selected from sucrose, guntianose, umbelliferose, lactose, planteose, isolignoses, α,α-trehalose, raffinose, lignoses, umbilicin, stachyose, verbascoses, and the like.

Examples of the polysaccharide may include at least one selected from cellulose, quince seed, chondroitinsulfate, starch, galactan, dermatan sulfate, glycogen, acasia gum, heparansulfate, hyaluronan, gum tragacanth, keratan sulfate, chondoroitin, xanthan gum, mucoitin sulfate, guar gum, dextran, keratosulfate, locust bean gum, succinoglycan, caronic acid, and the like.

Examples of other polyols may include at least one polyol selected from polyoxyethylene methyl glucoside (Glucam E-10), polyoxypropylene methyl glucoside (Glucam P-10), and the like.

Examples of the amphoteric surfactant that may be used may include: imidazoline-based amphoteric surfactant (such as sodium 2-undecyl-N,N,N-(hydroxyethylcarboxymethyl)-2-imidazoline and 2-cocoyl-2-imidazolinium hydroxide-1-carboxyethyloxy disodium salt); and betaine-based surfactant (such as 2-heptadecyl-N-carboxymethyl-N-hydroxyethyl imidazolinium betaine, lauryl dimethylaminoacetic acid betaine, alkyl betaine, amidobetaine, and sulfobetaine).

Examples of the cationic surfactants may include alkyltrimethyl ammonium salt (such as stearyltrimethyl ammonium chloride, lauryltrimethyl ammonium chloride); alkylpyridinium salt (such as cetylpyridinium chloride); distearyldimethyl ammonium chloride; dialkyldimethyl ammonium salt; poly (N,N'-dimethyl-3,5-methylenepiperidinium) chloride; alkyl quaternary ammonium salt; alkyldimethylbenzyl ammonium salt; alkylisoquinolinium salt; dialkylmorphonium salt; POE alkylamine; alkylamine salt; polyamine fatty acid derivative; amyl alcohol fatty acid derivative; benzalkonium chloride; benzethonium chloride, and the like.

The terms "powdery body" and "powder" as used herein are synonymous. Powdery bodies are not particularly limited so long as they are generally usable for makeup purposes, for example. Examples may include inorganic powder such as talc, kaolin, mica, sericite, muscovite, biotite, phlogopite, synthetic mica, silica, zeolite, barium sulfate, calcined calcium sulfate, calcined gypsum, calcium phosphate, fluorine apatite, hydroxyapatite, ceramic powder; inorganic white family pigment such as titanium dioxide, zinc oxide; inorganic red family pigment such as iron oxide (colcothar), iron titanate; inorganic brown family pigment such as γ-iron oxide; inorganic yellow family pigment such as yellow iron oxide, loess; inorganic black family pigment such as black iron oxide, carbon black, lower titanium oxide; inorganic purple family pigment such as manganese violet, cobalt violet; inorganic green family pigment such as chrome oxide, chrome hydroxide, cobalt titanate; inorganic blue family pigment such as ultramarine, iron blue; pearl pigment such as titanium oxide coated mica, titanium oxide coated bismuth oxychloride, titanium oxide coated talc, colored titanium oxide coated mica, bismuth oxychloride, argentine; metal powder pigment such as aluminum powder, copper powder; organic pigment of zirconium, barium, or aluminum lake and etc. such as Red No.202, Red No.205, Red No.220, Red No.228, Red No.405, Orange No.204, Yellow No.205, Yellow No.401, Blue No.404; organic pigment such as Red No.3, Red No.104, Red No.227, Red No.401, Orange No.205, Yellow No.4, Yellow No.202, Green No.3, and Blue No.1, natural pigment such as chlorophyll, β-carotene; and the like.

Examples of the moisturizers may include polyethylene glycol, propylene glycol, glycerin, 1,3-butylene glycol, xylitol, sorbitol, maltitol, chondroitin sulfate, hyaluronic acid, mucoitin sulfate, charonic acid, atelocollagen, cholesteryl 12-hydroxystearate, sodium lactate, bile salt, dl-pyrrolidone carboxylate, alkyleneoxide derivative, short-chain soluble collagen, diglycerin (EO)PO adduct, chestnut rose extract, yarrow extract, melilot extract, and the like.

Examples of the natural water-soluble polymer may include plant-based polymer (such as gum Arabic, gum tragacanth, galactan, guar gum, locust bean gum, gum karaya, carrageenan, pectine, agar, quince seed (cydonia oblonga), algae colloid (brown algae extract), starch (rice, corn, potato, wheat) ,glicyrrhizic acid); microorganism based polymer (such as xanthan gum, dextran, succinoglycan, pullulan, etc), animal-based polymer (such as collagen, casein, albumin, gelatine, etc) and the like.

Examples of the semisynthetic water-soluble polymer may include starch-based polymer (such as carboxymethyl starch, methylhydroxypropyl starch, etc); cellulose-based polymer (such as methylcellulose, ethylcellulose, methylhydroxypropylcellulose, hydroxyethylcellulose, cellulose sodium sulfate, hydroxypropylcellulose, carboxymethylcellulose, sodium calboxymethyl cellulose, crystalline cellulose, cellulose powder, etc); algin acid-based polymer (such as sodium alginate, propylene glycol alginate ester, etc), and the like.

Examples of the synthetic water-soluble polymer may include vinyl based polymer (such as polyvinyl alcohol, polyvinyl methyl ether, polyvinylpyrrolidone, carboxyvinylpolymer, etc); polyoxyethylene based polymer (such as polyoxyethylenepolyoxypropylene copolymer such as polyethylene glycol 20,000, 40,000 and 60,0000, etc); acrylic polymer (such as sodium polyacrylate, polyethylacrylate, polyacrylamide, etc); polyethyleneimine; cationic polymer; and the like.

Examples of the oily component that may be used include liquid oils, solid fats, waxes, hydrocarbons, higher fatty acids, and synthetic ester oils.

Examples of the liquid oil that may be used may include avocado oil, camellia oil, turtle oil, macadamia nut oil, corn oil, mink oil, olive oil, rapeseed oil, egg yolk oil, sesame oil, par chic oil, wheat germ oil, southern piece oil, castor oil, linseed oil, safflower oil, cotton seed oil, perilla oil, soybean oil, groundnut oil, brown real oil, torreya oil, rice bran oil, Chinese tung oil, Japanese tung oil, jojoba oil, germ oil, triglycerol, and the like.

Examples of the solid fat that may be used may include cacao butter, coconut oil, horse fat, hydrogenated coconut oil, palm oil, beef tallow, sheep tallow, hydrogenated beef tallow, palm kernel oil, lard, beef bones fat, Japan wax kernel oil, hardened oil, hoof oil, Japan wax, hydrogenated caster oil, and the like.

Examples of the waxes that may be used may include beeswax, candelilla wax, cotton wax, carnauba wax, bayberry wax, insect wax, spermaceti, montan wax, bran wax, lanolin, kapok wax, lanolin acetate, liquid lanolin, sugarcane wax, lanolin fatty acid isopropyl ester, hexyl laurate, reduced lanolin, jojoba wax, hardened lanolin, shellac wax, POE lanolin alcohol ether, POE lanolin alcohol acetate, POE cholesterol ether, lanolin fatty acid polyethylene glycol, POE hydrogenated lanolin alcohol ether, and the like.

Examples of the hydrocarbon oils that may be used may include liquid paraffin, ozocerite, squalane, pristane, paraffin, ceresin. squalene, vaseline, microcrystalline wax, and the like.

Examples of the higher fatty asid that may be used may include lauric acid, myristic acid, palmitic acid, stearic acid, behenic acid, oleic acid, undecylenic acid, tallic acid, isostearic acid, linoleic acid, linolenic acid, eicosapentaenoic acid(EPA), docosahexaenoic acid(DHA) and the like.

Examples of the synthesis ester oils that may be used may include isopropyl myristate, cetyl octanoate, octyldodecyl myristate, isopropyl palmitate, butyl stearate, hexyl laurate, myristyl myristate, decyl oleate, hexyldecyl dimethyl octanoate, cetyl lactate, myristyl lactate, lanolin acetate, isocetyl stearate, isocetyl isostearate, cholesteryl 12-hydroxy stearate, ethylene glycol di-2-ethyl hexanoate, di-penta erythritol fatty acid ester, N-alkyl glycol monoisostearate, neopentyl glycol dicaprate, diisostearyl malate, glyceryl di-2-heptyl undecanoate, trimethyrol propane tri-2-ethyl hexanoate, trimethyrol propane triisostearate, pentaerythritol tetra-2-ethyl hexanoate, glyceryl tri-2-ethyl hexanoate, glyceryl trioctanoate, glyceryl triisopalmitate, trimethyrol propane triisostearate, cetyl 2-ethylhexanoate, 2-ethylhexyl palmitate, glyceryl trimyristate, glyceride tri-2-heptyl undecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, triethyl citrate, and the like.

Examples of the film-forming agent may include an anionic film-forming agent (such as (meta)acrylic acid/(meta)acrylic acid ester copolymer, methyl vinyl ether/maleic anhydride coplymer, etc), a cationic film-forming agent (such as cationic cellulose, diallyldimethylammonium chloride polymer, diallyldimethylammonium chloride/acrylic amide copolymer, etc), a nonionc film-forming agent (such as polyvinyl alcohol, polyvinylpyrrolidone, polyvinyl acetate, polyacrylic ester copolymer, (meta)acrylamide, polymeric silicone, silicone resin, trimethylsiloxysilicate, etc), and the like.

Examples of the ultraviolet light absorbers may include benzoic acid family ultraviolet light absorber (such as p-aminobenzoic acid (hereinafter abbreviated as PABA), PABA monoglycerine ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA ethyl ester, etc); anthranilic acid family ultraviolet light absorber (such as homomenthyl N-acetylanthranilate etc); salicylic acid family ultraviolet light absorber (such as amyl salicylate, menthyl salicylate, homomenthyl salicylate, octyl salicylate, phenyl salicylate, benzyl salicylate, p-isopropanolphenyl salicylate, etc); cinnamic acid family ultraviolet light absorber (such as octyl methoxycinnamate, ethyl 4-isopropylcinnamate, methyl 2,5-diisopropylcinnamate, ethyl 2,4-diisopropylcinnamate, methyl 2,4-diisopropylcinnamate, propyl p-methoxycinnamate, isopropyl p-methoxycinnamate, isoamyl p-methoxycinnamate, octyl p-methoxycinnamate (2-ethylhexyl p-methoxycinnamate), 2-ethoxyethyl p-methoxycinnamate, cyclohexyl p-methoxycinnamate, ethyl α-cyano-β-phenylcinnamate, 2-ethylhexyl α-cyano-β-phenylcinnamate, glyceryl mono-2-ethylhexanoyl-diparamethoxy cinnamate, etc); benzophenone family ultraviolet light absorber (such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, 4-hydroxy-3-carboxybenzophenone, etc); 3-(4'-methylbenzylidene)-d,l-camphor and 3-benzylidene-d,l-camphor; 2-phenyl-5-methylbenzoxazol; 2,2'-hydroxy-5-methylphenylbenzotriazol, 2-(2'-hydroxy-5'-t-octylphenyl) benzotriazol, 2-(2'-hydroxy-5'-methylphenylbenzotriazol; dibenzalazine; dianisoylmethane; 4-methoxy-4'-t-butyldibenzoylmethane; 5-(3,3-dimethyl-2-norbornylidene)-3-pentane-2-one; dimorpholinopyridazinone; 2-ethylhexyl 2-cyano-3,3-diphenylacrylate; 2,4-bis-{[4-(2-ethylhexyloxy)-2-hydroxy]-phenyl}-6-(4-methoxyphenyl)-(1,3,5)-tria zine, and the like.

Examples of the metal ion sequestrant may include 1-hydroxyethane-1, 1-diphosphonic acid, 1-hydroxyethane, 1-diphosphonic acid 4Na salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, trisodium hydroxyethyl ethylenediamine triacetate, and the like.

Examples of the amino acid may include neutral amino acid (such as threonine, cysteine, etc); basic amino acid (such as hydroxylysine, etc) and the like. Examples of the amino acid derivative may include sodium acyl sarcosinate (sodium lauroyl sarcosinate), acyl glutamate, sodium acyl β-alanine, glutathione, pyrrolidone carboxylate, and the like.

Examples of the organic amine may include monoethanolamine, diethanolamine, triethanolamine, morpholine, triisopropanolamine, 2-amino-2-methyl-1,3-propanediol, 2-amino-2-methyl-1-propanol, and the like.

Examples of the polymer emulsion may include acrylic resin emulsion, ethyl polyacrylate emulsion, solution of acrylic resin, polyacrylalkylester emulsion, polyvinyl acetate resin emulsion, natural rubber latex, and the like.

For example, the silicone elastomers may include cross-linked silicone in which silicone polymers are three-dimensionally crosslinked. Examples of silicone elastomers may include dimethicone crosspolymer, dimethicone/vinyl dimethicone crosspolymer, dimethicone/phenyl vinyl dimethicone crosspolymer, vinyl dimethicone/lauryl dimethicone crosspolymer, lauryl polydimethyl siloxyethyl dimethicone/bis-vinyl dimethicone crosspolymer, alkyl (C₃₀₋₄₅) cetearyl dimethicone crosspolymer, and cetearyl dimethicone crosspolymer.

Examples of the pH modifier may include buffer such as lactic acid-sodium lactate, citric acid-sodium citrate, succinic acid-sodium succinate, and the like.

Examples of the vitamins may include vitamine A, B1, B2, B6, C, E and derivatives thereof, pantothenic acid and derivatives thereof, biotin, and the like.

Examples of the anti-oxidant may include tocopherols, dibutyl hydroxy toluene, butyl hydroxy anisole, and gallic acid esters, and the like.

Examples of the anti-oxidant aid may include phosphoric acid, citric acid, ascorbic acid, maleic acid, malonic acid, succinic acid, fumaric acid, cephalin, hexamethaphosphate, phytic acid, ethylenediaminetetraacetic acid, and the like.

Examples of other containable compositions may include an antiseptic agent (such as ethylparaben, butylparaben, chlorphenesin, 2-phenoxyethanol, etc); antiphlogistic (such as glycyrrhizinic acid derivatives, glycyrrhetic acid derivatives, salicylic acid derivatives, hinokitiol, zinc oxide, allantoin, etc); a skin-whitening agent (such as placental extract, saxifrage extract, arbutin, etc); various extracts (such as phellodendron bark (cork tree bark), coptis rhizome, lithospermum, peony, swertia herb, birch, sage, loquat, carrot, aloe, mallow, iris, grape, coix seed, sponge gourd, lily, saffron, cnidium rhizome, ginger, hypericum, restharrow, garlic, red pepper, citrus unshiu, Japanese angelica, seaweed, etc); an activator (such as royal jelly, photosenstizer, cholesterol derivatives, etc); a blood circulation promotion agent (such as nonylic acid vanillylamide, nicotine acid benzyl ester, nicotine acid β-butoxyethyl ester, capsaicin, zingerone, cantharides tincture, ichthammol, tannic acid, α-borneol, tocopheryl nicotinate, meso-inositol hexanicotinate, cyclandelate, cinnarizine, tolazoline, acetylcholine, verapamil, cepharanthine, γ-oryzanol, etc); an antiseborrheric agent, (such as sulfur, thianthl, etc); an anti-inflammatory agent (such as tranexamic acid, thiotaurine, hypotaurine, etc), and the like.

The composition of the present disclosure further may inculde, as necessary, caffeine, tannin, verapamil, tranexamic acid and derivatives thereof; various crude drug extracts such as licorice, Chinese quince, Pyrola japonica and the like; drugs such as tocopherol acetate, glycyrrhetinic acid, glycyrrhizic acid and derivatives thereof, or salts thereof; skin-whitening agents such as vitamin C, magnesium ascorbyl phosphate, ascorbic acid glucoside, arbutin, kojic acid and the like; amino acids such as arginine and lysine and the like and derivatives thereof.

In the composition of the present disclosure, from the viewpoint of emulsion stability, the average particle size of emulsion particles is preferably less than 5 µm, more preferably 3 µm or less, at room temperature (RT).

The hardness of the composition of the present disclosure is preferably 8 or greater, more preferably 10 or greater. The hardness of the composition of the present disclosure is preferably 22 or less, more preferably 20 or less. When the hardness is within the aforementioned range, the composition can be scooped up with the finger just by lightly touching the composition with the finger. Also, moistness arises when the composition is applied to the skin, and swift finish can be achieved upon application. The hardness can be measured at 25°C with a rheometer (needle diameter: 11.3 ø; load: 200 g; penetration depth: 10.0 mm; penetration speed: 300 mm/min).

With the composition of the present disclosure, the user can obtain, upon use, a smooth and pleasant feel when applying the composition to the skin. Also, after application, the user can feel excellent moistness.

The composition of the present disclosure can maintain a stable emulsion state even when an active ingredient potentially of improving skin such as an organic acid is included. Further, since the content of the active ingredient can be increased, the effects brought about by the active ingredient can be enhanced.

Next, methods for producing the oil-in-water emulsion composition of the present disclosure will be described. The composition of the present disclosure can be prepared according to generally known methods, without being limited to a specific method. For example, the oil-in-water emulsion composition can be prepared by adding an oil phase, in which oil-soluble components have been dissolved, to a water phase, in which water-soluble components have been dissolved, and stirring the two phases.

### Examples

Examples of the composition of the present disclosure will be described below. The composition of the present disclosure, however, is not limited to the following examples. Further, the composition of the present disclosure is not limited to cosmetics. The unit employed for indicating the content by percentage shown in the Tables is percent by mass (mass%). The evaluation items described in the Tables were evaluated according to the criteria described below.

The method for producing each composition is described. An oil phase was prepared by dissolving oil-soluble components, such as the component (A), the component (C), and the component (F), in an oily component (a portion of the component (G)) at high temperatures. On the other hand, a water phase was prepared by dissolving water-soluble components, such as the component (B), the component (D), and the component (E), in an aqueous solvent (a portion of the component (G)) such as purified water at high temperatures. The oil phase was added to the water phase and the two phases were stirred and then cooled, to obtain an oil-in-water emulsion composition.

### Feel upon Use:

Ten evaluators applied each composition of the respective Test Examples to the skin, and the feel upon use was evaluated according to the number of evaluators who were able to actually feel "skin smoothness (soft feeling)", "moistness", and "swift finish".
A: 10 evaluators.
B: 7 to 9 evaluators.
C: 3 to 6 evaluators.
D: 0 to 2 evaluators.

The overall evaluation was found according to the following criteria by calculating the average number of evaluators who were able to actually perceive the feelings upon use according to the respective evaluation items.
A: 8 evaluators or more.
B: 6 to 7 evaluators.
C: 4 to 5 evaluators.
D: 3 evaluators or fewer.

### Emulsion Stability:

Each composition immediately after emulsification was observed with a microscope, and the average particle size of emulsion particles was measured. Emulsion stability was evaluated according to the average particle size of the emulsion particles.
A: 3 µm or less.
B: Greater than 3 µm to less than 5 µm.
C: 5 µm or greater.

### Hardness (11.3 ø):

The hardness was measured according to the method described above.
A: 10 or greater to less than 20.
B: 8 or greater to less than 10, or 20 or greater to less than 22.
C: Less than 8, or 22 or greater.

### Test Examples 1 to 3:

The formulations and evaluation results of the compositions according to the respective Test Examples 1 to 3 are shown in Table 1. In Test Example 1, high emulsion stability was obtained even without containing an anionic surfactant (B) indicated as component (2). In Test Example 2, however, increasing the content by percentage of piperidinepropionic acid (D) indicated as component (5), which is an organic acid, caused deterioration in emulsion stability. It is considered that the component (D) negatively affected emulsion stability. So, in Test Example 3, an anionic surfactant (B), indicated as component (2), was further added to the composition of Test Example 2; in this way, it was possible to improve emulsion stability as in Test Example 1, and also, the evaluation regarding the feel upon use of the composition was equivalent to that of Test Example 1. It was thus found that, in cases where emulsion stability is inhibited by the component (D), emulsion stability can be improved by adding the component (B). From Test Example 3, it is considered that the content by percentage of the component (B) is preferably 0.03% by mass or greater, more preferably 0.04% by mass or greater.

**[Table 1]**

| | Test Example | | 1 | 2 | 3 |
|---|---|---|---|---|---|
| (1) | (A) POE (20) behenyl ether *1 | | 0.5 | 0.5 | 0.5 |
| (2) | (B) Sodium methyl stearoyl taurate | | - | - | 0.05 |
| (3) | (C) Stearyl alcohol | | 1 | 1 | 1 |
| (4) | (C) Behenyl alcohol | | 1.5 | 1.5 | 1.5 |
| (5) | (D) Piperidinepropionic acid | | 1 | 3 | 3 |
| (6) | (E) Dimethylacrylamide/sodium acryloyldimethy ltaurate crosspolymer | | 0.5 | 0.5 | 0.5 |
| (7) | (E) Xanthan gum | | 0.05 | 0.05 | 0.05 |
| (8) | (F) Sorbitan tristearate *2 | | 0.1 | 0.1 | 0.1 |
| (9) | (F) Self-emulsifying glyceryl monostearate | | 1.0 | 1.0 | 1.0 |
| (10) | (G) Glycerin | | 5 | 5 | 5 |
| (11) | (G) 1,3-Butylene glycol | | 5 | 5 | 5 |
| (12) | (G) Dipropylene glycol | | 7 | 7 | 7 |
| (13) | (G) Liquid paraffin | | 2 | 2 | 2 |
| (14) | (G) Methylpolysiloxane | | 5 | 5 | 5 |
| (15) | (G) Pentaerythritol tetra 2-ethylhexanoate *3 | | 1 | 1 | 1 |
| (16) | (G) Ion-exchanged water | | Balance | Balance | Balance |
| Total | | | 100 | 100 | 100 |
| Amount of substance of Component (D) (mmol/100g) | | | 6.3 | 19.1 | 19.1 |
| Component (B)/(Component (A)+Component (F)) | | | 0 | 0 | 0.031 |
| Evalua tion | Feel upon use | Skin smoothness | B | - | B |
| | | Moistness | A | - | A |
| | | Swift finish | A | - | A |
| | | Overall | A | - | A |
| | Emulsion stability | | A | C | A |
| | Hardness (measurement value) | | A(15) | A(15) | A(14) |
| *1: NIKKOL® BB-20 from Nikko Chemicals Co., Ltd. | | | | | |
| *2: NIKKOL® SS-30V from Nikko Chemicals Co., Ltd. | | | | | |
| *3: RA-PE-408 from Nippon Fine Chemical Co., Ltd. | | | | | |

### Test Examples 4 to 10:

The formulations and evaluation results of the compositions according to the respective Test Examples 4 to 10 are shown in Table 2. In Test Examples 4 to 10, the content by percentage of the component (B), i.e., the anionic surfactant, was varied.

In Test Example 4, the amount of the component (B) added was 0.5% by mass. The result was that, although emulsion stability was attained, skin smoothness and swift finish deteriorated. By reducing the content by percentage of the component (B), however, it was possible to improve the feel upon use while maintaining emulsion stability. Thus, the content by percentage of the component (B) is preferably less than 0.5% by mass, more preferably 0.4% by mass or less, even more preferably 0.3% by mass or less, even more preferably 0.2% by mass or less, even more preferably 0.1% by mass or less.

In Test Example 10 in which the content by percentage of the component (B) was 0.02% by mass, the emulsion-impairing action by the component (D) was intensified, thus making the emulsion state unstable. Hardness also deteriorated. Thus, the content by percentage of the component (B) is preferably greater than 0.02% by mass, more preferably 0.03% by mass or greater.

**[Table 2]**

| | Test Example | | 4 | 5 | 6 | 7 | 8 | 9 | 10 |
|---|---|---|---|---|---|---|---|---|---|
| (1) | (A) POE (20) behenyl et her *1 | | 0.5 | 0.5 | 0.5 | 0.5 | 0.5 | 0.5 | 0.5 |
| (2) | (B) Sodium methyl stear oyl taurate | | 0.5 | 0.3 | 0.2 | 0.1 | 0.05 | 0.035 | 0.02 |
| (3) | (C) Stearyl alcohol | | 1 | 1 | 1 | 1 | 1 | 1 | 1 |
| (4) | (C) Behenyl alcohol | | 1.5 | 1.5 | 1.5 | 1.5 | 1.5 | 1.5 | 1.5 |
| (5) | (D) Piperidinepropionic a cid | | 3 | 3 | 3 | 3 | 3 | 3 | 3 |
| (6) | (E) Dimethylacrylamide/s odium acryloyldimethylta urate crosspolymer | | 0.5 | 0.5 | 0.5 | 0.5 | 0.5 | 0.5 | 0.5 |
| (7) | (E) Xanthan gum | | 0.05 | 0.05 | 0.05 | 0.05 | 0.05 | 0.05 | 0.05 |
| (8) | (F) Sorbitan tristearate * 2 | | 0.1 | 0.1 | 0.1 | 0.1 | 0.1 | 0.1 | 0.1 |
| (9) | (F) Self-emulsifying glyce ryl monostearate | | 1.0 | 1.0 | 1.0 | 1.0 | 1.0 | 1.0 | 1.0 |
| (10) | (G) Glycerin | | 5 | 5 | 5 | 5 | 5 | 5 | 5 |
| (11) | (G) 1,3-Butylene glycol | | 5 | 5 | 5 | 5 | 5 | 5 | 5 |
| (12) | (G) Dipropylene glycol | | 7 | 7 | 7 | 7 | 7 | 7 | 7 |
| (13) | (G) Liquid paraffin | | 2 | 2 | 2 | 2 | 2 | 2 | 2 |
| (14) | (G) Methylpolysiloxane | | 5 | 5 | 5 | 5 | 5 | 5 | 5 |
| (15) | (G) Pentaerythritol tetra 2-ethylhexanoate *3 | | 1 | 1 | 1 | 1 | 1 | 1 | 1 |
| (16) | (G) Ion-exchanged water | | Balance | Balance | Balance | Balance | Balance | Balance | Balance |
| Total | | | 100 | 100 | 100 | 100 | 100 | 100 | 100 |
| Amount of substance of Compon ent (D) (mmol/100g) | | | 19.1 | 19.1 | 19.1 | 19.1 | 19.1 | 19.1 | 19.1 |
| Component (B)/(Component (A)+ Component (F)) | | | 0.31 | 0.19 | 0.13 | 0.063 | 0.031 | 0.022 | 0.013 |
| Evaluation | Feel upon use | Skin smoothness | C | B | B | B | B | B | - |
| | | Moistness | B | B | B | A | A | A | - |
| | | Swift finish | D | C | B | A | A | A | - |
| | | Overall | C | B | B | A | A | A | - |
| | Emulsion stability | | A | A | A | A | A | A | C |
| | Hardness (measurement value) | | A(13) | A(10) | A(12) | A(13) | A(14) | A(15) | B(8) |

### Test Examples 11 to 17:

The formulations and evaluation results of the compositions according to the respective Test Examples 11 to 17 are shown in Table 3. In Test Examples 11 to 15, potassium 4-methoxysalicylate, which is an organic acid salt, and a citrate buffer were added instead of piperidinepropionic acid which was the component (D) in Test Examples 1 to 10, and the content of potassium 4-methoxysalicylate was varied. In Test Examples 16 and 17, L-ascorbic acid 2-glucoside, which is an organic acid derivative, was added as the component (D). Test Examples 12, 14, and 17, which did not contain the component (B), were unable to attain emulsion stability. In contrast, Test Examples 11, 13, 15, and 16, which contained the component (B), were able to attain emulsion stability. It was thus found that organic acid salts and organic acid derivatives, and not only organic acids, impair emulsion stability. It was also found that, even in cases where organic acid salts and derivatives impair emulsion stability, adding the component (B) can improve emulsion stability.

Test Examples 1 to 10 employed a polyoxyethylene alkyl ether as the component (A), i.e., the hydrophilic nonionic surfactant, whereas Test Example 15 employed a polyalkylene glycol fatty acid ester. The result was that Test Example 15 was also able to attain emulsion stability and a desired hardness. It was thus found that polyalkylene glycol fatty acid esters are also useful against emulsion stability impairment caused by the component (D).

**[Table 3]**

| | Test Example | 11 | 12 | 13 | 14 | 15 | 16 | 17 |
|---|---|---|---|---|---|---|---|---|
| (1) | (A) POE (20) behenyl ether *1 | 0.5 | 0.5 | 0.5 | 0.5 | - | 0.5 | 0.5 |
| (2) | (A) Polyethylene glycol monostearate (40E.O.) *4 | - | - | - | - | 0.5 | | |
| (3) | (B) Sodium methyl stearoyl taurate | 0.1 | - | 0.1 | - | 0.1 | 0.1 | - |
| (4) | (C) Stearyl alcohol | 1 | 1 | 1 | 1 | 1 | 1 | 1 |
| (5) | (C) Behenyl alcohol | 1.5 | 1.5 | 1.5 | 1.5 | 1.5 | 1.5 | 1.5 |
| (6) | (D) Potassium 4-methoxysalicylate | 1 | 1 | 3 | 3 | 2 | - | - |
| (7) | (D) L-ascorbic acid 2-glucoside | - | - | - | - | - | 2 | 2 |
| (8) | (D) Citric acid | 0.01 | 0.01 | 0.01 | 0.01 | 0.01 | 0.01 | 0.01 |
| (9) | (D) Sodium citrate | 0.09 | 0.09 | 0.09 | 0.09 | 0.09 | 0.09 | 0.09 |
| (10) | (E) Dimethylacrylamide/sodium acryloyldimethyltaurate cross polymer | 0.5 | 0.5 | 0.5 | 0.5 | 0.5 | 0.5 | 0.5 |
| (11) | (E) Xanthan gum | 0.05 | 0.05 | 0.05 | 0.05 | 0.05 | 0.05 | 0.05 |
| (12) | (F) Sorbitan tristearate *2 | 0.1 | 0.1 | 0.1 | 0.1 | 0.1 | 0.1 | 0.1 |
| (13) | (F) Self-emulsifying glyceryl monostearate | 1.0 | 1.0 | 1.0 | 1.0 | 1.0 | 1.0 | 1.0 |
| (14) | (G) Glycerin | 5 | 5 | 5 | 5 | 5 | 5 | 5 |
| (15) | (G) 1,3-Butylene glycol | 5 | 5 | 5 | 5 | 5 | 5 | 5 |
| (16) | (G) Dipropylene glycol | 7 | 7 | 7 | 7 | 7 | 7 | 7 |
| (17) | (G) Liquid paraffin | 2 | 2 | 2 | 2 | 2 | 2 | 2 |
| (18) | (G) Methylpolysiloxane | 5 | 5 | 5 | 5 | 5 | 5 | 5 |
| (19) | (G) Pentaerythritol tetra 2-ethylhexanoate *3 | 1 | 1 | 1 | 1 | 1 | 1 | 1 |
| (20) | (G) Ion-exchanged water | Balance | Balance | Balance | Balance | Balance | Balance | Balance |
| Total | | 100 | 100 | 100 | 100 | 100 | 100 | 100 |
| Amount of substance of component (D) (mmol/100g) | | 5.2 | 5.2 | 14.9 | 14.9 | 10.1 | 6.3 | 6.3 |
| Component (B)/(Component (A)+Component (F)) | | 0.06 | 0 | 0.06 | 0 | 0.06 | 0.06 | 0 |
| Evaluation | Emulsion stability | B | C | B | C | A | A | C |
| | Hardness (measurement value) | A(19) | A(13) | A(17) | A(14) | A(15) | A(13) | A(18) |
| *4: NIKKOL® MYS-40V from Nikko Chemicals Co., Ltd. | | | | | | | | |

### Test Examples 18 to 21:

The formulations and evaluation results of the compositions according to the respective Test Examples 18 to 21 are shown in Table 4. In Test Examples 18 to 21, carnosine or tranexamic acid, as other types of organic acids, was used instead of piperidinepropionic acid used in Test Examples 1 to 10. The result was that, similar to Test Examples 11 to 17, Test Examples 19 and 21, which did not contain the component (B), were unable to attain emulsion stability. Hardness also deteriorated in Test Example 19. In contrast, in Test Examples 18 and 20 which contained the component (B), it was possible to attain emulsion stability and also improve hardness. It was thus found that emulsion stability and hardness may deteriorate even with other organic acids. It was also found that, even in cases where organic acids impair emulsion stability, adding the component (B) can improve emulsion stability and hardness.

**[Table 4]**

| | Test Example | 18 | 19 | 20 | 21 |
|---|---|---|---|---|---|
| (1) | (A) POE (20) behenyl ether *1 | 0.5 | 0.5 | 0.5 | 0.5 |
| (2) | (B) Sodium methyl stearoyl taurate | 0.1 | - | 0.1 | - |
| (3) | (C) Stearyl alcohol | 1 | 1 | 1 | 1 |
| (4) | (C) Behenyl alcohol | 1.5 | 1.5 | 1.5 | 1.5 |
| (5) | (D) Carnosine | 3.5 | 3.5 | - | - |
| (6) | (D) Tranexamic acid | - | - | 2 | 2 |
| (7) | (D) Citric acid | 0.01 | 0.01 | 0.01 | 0.01 |
| (8) | (D) Sodium citrate | 0.09 | 0.09 | 0.09 | 0.09 |
| (9) | (E) Dimethylacrylamide/sodium acryloyldimethyltaurate crosspolymer | 0.5 | 0.5 | 0.5 | 0.5 |
| (10) | (E) Xanthan gum | 0.05 | 0.05 | 0.05 | 0.05 |
| (11) | (F) Sorbitan tristearate *2 | 0.1 | 0.1 | 0.1 | 0.1 |
| (12) | (F) Self-emulsifying glyceryl monostearate | 1.0 | 1.0 | 1.0 | 1.0 |
| (13) | (G) Glycerin | 5 | 5 | 5 | 5 |
| (14) | (G) 1,3-Butylene glycol | 5 | 5 | 5 | 5 |
| (15) | (G) Dipropylene glycol | 7 | 7 | 7 | 7 |
| (16) | (G) Liquid paraffin | 2 | 2 | 2 | 2 |
| (17) | (G) Methylpolysiloxane | 5 | 5 | 5 | 5 |
| (18) | (G) Pentaerythritol tetra 2-ethylhexanoate *3 | 1 | 1 | 1 | 1 |
| (19) | (G) Ion-exchanged water | Balance | Balance | Balance | Balance |
| Total | | 100 | 100 | 100 | 100 |
| Amount of substance of component (D) (mmol/100g) | | 15.9 | 15.9 | 13.1 | 13.1 |
| Component (B)/(Component (A)+Component (F)) | | 0.06 | 0 | 0.06 | 0 |
| Evaluation | Emulsion stability | A | C | A | C |
| | Hardness (measurement value) | A(12) | B(9) | A(15) | A(13) |

### Test Examples 22 to 25:

The formulations and evaluation results of the compositions according to the respective Test Examples 22 to 25 are shown in Table 5. In Test Examples 22 to 24, the content of the component (A), i.e., the hydrophilic nonionic surfactant, was varied. Test Example 22 is identical to Test Example 8. All of the compositions were able to achieve excellent emulsion stability and hardness. It is thus considered that the content of the component (A) is preferably at least 0.4% by mass or greater. It is also considered that the content of the component (A) is preferably at most 0.7% by mass or less.

Test Example 25 used a surfactant in which the length of the polyoxyethylene chain was different from that of the component (A), i.e., the hydrophilic nonionic surfactant, used in Test Examples 1 to 24. Test Example 25 was also able to achieve results comparable to Test Example 7. Thus, the average number of moles of polyoxyethylene groups added in the polyoxyethylene alkyl ether is preferably at least 15 or greater. Further, the average number of moles of polyoxyethylene groups added is preferably at most 40 or less, more preferably 35 or less.

**Table 5**

| | Test Example | 22 | 23 | 24 | 25 |
|---|---|---|---|---|---|
| (1) | (A) POE (20) behenyl ether *1 | 0.5 | 0.55 | 0.6 | - |
| (2) | (A) POE (30) behenyl ether ^{*}5 | - | - | - | 0.5 |
| (3) | (B) Sodium methyl stearoyl taurate | 0.05 | 0.05 | 0.05 | 0.1 |
| (4) | (C) Stearyl alcohol | 1 | 1 | 1 | 1 |
| (5) | (C) Behenyl alcohol | 1.5 | 1.5 | 1.5 | 1.5 |
| (6) | (D) Piperidinepropionic acid | 3 | 3 | 3 | 3 |
| (7) | (D) Citric acid | 0.01 | 0.01 | 0.01 | 0.01 |
| (8) | (D) Sodium citrate | 0.09 | 0.09 | 0.09 | 0.09 |
| (9) | (E) Dimethylacrylamide/sodium acryloyldimethyltaurate crosspolymer | 0.5 | 0.5 | 0.5 | 0.5 |
| (10) | (E) Xanthan gum | 0.05 | 0.05 | 0.05 | 0.05 |
| (11) | (F) Sorbitan tristearate *2 | 0.1 | 0.1 | 0.1 | 0.1 |
| (12) | (F) Self-emulsifying glyceryl monostearate | 1.0 | 1.0 | 1.0 | 1.0 |
| (13) | (G) Glycerin | 5 | 5 | 5 | 5 |
| (14) | (G) 1,3-Butylene glycol | 5 | 5 | 5 | 5 |
| (15) | (G) Dipropylene glycol | 7 | 7 | 7 | 7 |
| (16) | (G) Liquid paraffin | 2 | 2 | 2 | 2 |
| (17) | (G) Methylpolysiloxane | 5 | 5 | 5 | 5 |
| (18) | (G) Pentaerythritol tetra 2-ethylhexanoate *3 | 1 | 1 | 1 | 1 |
| (19) | (G) Ion-exchanged water | Balance | Balance | Balance | Balance |
| Total | | 100 | 100 | 100 | 100 |
| Amount of substance of component (D) (mmol/100g) | | 19.5 | 19.5 | 19.5 | 19.5 |
| Component (B)/(Component (A)+Component (F)) | | 0.031 | 0.030 | 0.029 | 0.063 |
| Evaluation | Emulsion stability | A | A | A | A |
| | Hardness (measurement value) | A(14) | A(15) | A(17) | A(11) |
| *5: NIKKOL® BB-30 from Nikko Chemicals Co., Ltd. | | | | | |

### Test Examples 26 and 27:

The formulations and evaluation results of the compositions according to the respective Test Examples 26 and 27 are shown in Table 6. Test Examples 1 to 25 used a N-acyl methyl taurine salt for the component (B), i.e., the anionic surfactant. Test Example 26 used a N-acyl glutamic acid salt for the component (B). Test Example 27 used a phosphate ester salt. Both Test Examples 26 and 27 were able to achieve desired emulsion stability and hardness. It was thus found that, in addition to N-acyl methyl taurine salts, N-acyl glutamic acid salts and phosphate ester salts are useful as the component (B) against deterioration in emulsion stability and deterioration in hardness caused by the component (D).

**[Table 6]**

| | Test Example | 26 | 27 |
|---|---|---|---|
| (1) | (A) POE (20) behenyl ether *1 | 0.5 | 0.5 |
| (2) | (B) Sodium N-stearoyl-L-glutamate | 0.1 | - |
| (3) | (B) Potassium cetyl phosphate | - | 0.1 |
| (4) | (C) Stearyl alcohol | 1 | 1 |
| (5) | (C) Behenyl alcohol | 1.5 | 1.5 |
| (6) | (D) Piperidinepropionic acid | 3 | 3 |
| (7) | (D) Citric acid | 0.01 | 0.01 |
| (8) | (D) Sodium citrate | 0.09 | 0.09 |
| (9) | (E) Dimethylacrylamide/sodium acryloyldimethyltaurate crosspolymer | 0.5 | 0.5 |
| (10) | (E) Xanthan gum | 0.05 | 0.05 |
| (11) | (F) Sorbitan tristearate *2 | 0.1 | 0.1 |
| (12) | (F) Self-emulsifying glyceryl monostearate | 1.0 | 1.0 |
| (13) | (G) Glycerin | 5 | 5 |
| (14) | (G) 1,3-Butylene glycol | 5 | 5 |
| (15) | (G) Dipropylene glycol | 7 | 7 |
| (16) | (G) Liquid paraffin | 2 | 2 |
| (17) | (G) Methylpolysiloxane | 5 | 5 |
| (18) | (G) Pentaerythritol tetra 2-ethylhexanoate *3 | 1 | 1 |
| (19) | (G) Ion-exchanged water | Balance | Balance |
| Total | | 100 | 100 |
| Amount of substance of component (D) (mmol/100g) | | 19.5 | 19.5 |
| Component (B)/(Component (A)+Component (F)) | | 0.063 | 0.063 |
| Evaluation | Emulsion stability | A | A |
| | Hardness (measurement value) | B(8) | A(15) |

### Test Examples 28 and 29:

The formulations and evaluation results of the compositions according to the respective Test Examples 28 and 29 are shown in Table 7. In Test Examples 28 and 29, the content of the component (C), i.e., the higher alcohol, was varied compared to Test Examples 1 to 17. Both Test Examples 28 and 29 were also able to achieve desired emulsion stability and hardness. Thus, the content of the component (C) is preferably 0.5% by mass or greater, more preferably 0.8% by mass or greater. Further, the content of the component (C) is preferably 6% by mass or less, more preferably 5% by mass or less, even more preferably 4% by mass or less.

**[Table 7]**

| | Test Example | 28 | 29 |
|---|---|---|---|
| (1) | (A) POE (20) behenyl ether *1 | 0.5 | 0.5 |
| (2) | (B) Sodium methyl stearoyl taurate | 0.1 | 0.1 |
| (3) | (C) Stearyl alcohol | 0.3 | 1.5 |
| (4) | (C) Behenyl alcohol | 0.5 | 2.5 |
| (5) | (D) Piperidinepropionic acid | 3 | 3 |
| (6) | (D) Citric acid | 0.01 | 0.01 |
| (7) | (D) Sodium citrate | 0.09 | 0.09 |
| (8) | (E) Dimethylacrylamide/sodium acryloyldimet hyltaurate crosspolymer | 0.5 | 0.5 |
| (9) | (E) Xanthan gum | 0.05 | 0.05 |
| (10) | (F) Sorbitan tristearate *2 | 0.1 | 0.1 |
| (11) | (F) Self-emulsifying glyceryl monostearate | 1.0 | 1.0 |
| (12) | (G) Glycerin | 5 | 5 |
| (13) | (G) 1,3-Butylene glycol | 5 | 5 |
| (14) | (G) Dipropylene glycol | 7 | 7 |
| (15) | (G) Liquid paraffin | 2 | 2 |
| (16) | (G) Methylpolysiloxane | 5 | 5 |
| (17) | (G) Pentaerythritol tetra 2-ethylhexanoate *3 | 1 | 1 |
| (18) | (G) Ion-exchanged water | Balance | Balance |
| Total | | 100 | 100 |
| Amount of substance of component (D) (mmol/100g) | | 19.5 | 19.5 |
| Component (B)/(Component (A)+Component (F)) | | 0.063 | 0.063 |
| Evaluation | Emulsion stability | A | A |
| | Hardness (measurement value) | B(8) | A(15) |

Formulation examples of the oil-in-water emulsion composition of the present disclosure are described below. The application examples of the oil-in-water emulsion composition of the present disclosure, however, are not limited by the following formulation examples.

### Formulation Example 1: Sunblock skincare cream (Table 8)

**[Table 8]**

| | Components | Content (mass%) |
|---|---|---|
| (1) | (A) POE (20) behenyl ether | 1.5 |
| (2) | (B) Sodium methyl stearoyl taurate | 0.3 |
| (3) | (C) Stearyl alcohol | 1 |
| (4) | (C) Behenyl alcohol | 3 |
| (5) | (D) L-ascorbic acid 2-glucoside | 2 |
| (6) | (E) Sodium acrylate/sodium acryloyldimethyltaurate copolymer | 1 |
| (7) | (F) Sorbitan tristearate | 0.2 |
| (8) | (G) Glycerin | 10 |
| (9) | (G) 1,3-Butylene glycol | 10 |
| (10) | (G) Dipropylene glycol | 5 |
| (11) | (G) Xylitol | 1 |
| (12) | (G) Hydrogenated polydecene | 10 |
| (13) | (G) Polybutylene glycol/PPG-9/1 copolymer | 2 |
| (14) | (G) Petrolatum | 2 |
| (15) | (G) Hydrogenated palm oil | 2 |
| (16) | (G) Dimethicone | 3 |
| (17) | (G) Octocrylene | 5 |
| (18) | (G) Butyl methoxydibenzoylmethane | 2.5 |
| (19) | (G) Dipotassium glycyrrhizate | 0.1 |
| (20) | (G) Chelating agent | q.s. |
| (21) | (G) Antiseptic agent | q.s. |
| (22) | (G) Ion-exchanged water | Balance |
| Total | | 100 |
| Component (B)/(Component (A)+Component (F)) | | 0.18 |

### Formulation Example 2: Hand cream (Table 9)

**[Table 9]**

| | Components | Content (mass%) |
|---|---|---|
| (1) | (A) POE (20) behenyl ether *1 | 0.3 |
| (2) | (A) Polyethylene glycol monostearate (40E.O.) | 0.7 |
| (3) | (B) Sodium methyl stearoyl taurate | 0.3 |
| (4) | (C) Stearyl alcohol | 1 |
| (5) | (C) Behenyl alcohol | 3 |
| (6) | (D) Tranexamic acid | 2 |
| (7) | (D) Citric acid | 0.04 |
| (8) | (D) Sodium citrate | 0.06 |
| (9) | (E) Sodium acrylate/sodium acryloyldimethyltaurate copolymer | 0.5 |
| (10) | (F) Sorbitan tristearate | 1 |
| (11) | (F) Self-emulsifying glyceryl monostearate | 0.5 |
| (12) | (G) Glycerin | 10 |
| (13) | (G) 1,3-Butylene glycol | 10 |
| (14) | (G) Triisostearin | 1 |
| (15) | (G) Pentaerythrityl tetraethylhexanoate | 10 |
| (16) | (G) Myristyl myristate | 2 |
| (17) | (G) Dimethicone | 5 |
| (18) | (G)V-A Acetate | 2.5 |
| (19) | (G) Chelating agent | q.s. |
| (20) | (G) Antiseptic agent | q.s. |
| (21) | (G) Ion-exchanged water | Balance |
| Total | | 100 |
| Component (B)/(Component (A)+Component (F)) | | 0.12 |

### Formulation Example 3: Anti-aging cream (Table 10)

**[Table 10]**

| | Components | Content (mass%) |
|---|---|---|
| (1) | (A) Polyethylene glycol monostearate (40E.O.) | 1.5 |
| (2) | (B) Potassium cetyl phosphate | 0.2 |
| (3) | (C) Stearyl alcohol | 0.6 |
| (4) | (C) Behenyl alcohol | 0.8 |
| (5) | (D) Kojic acid | 1 |
| (6) | (D) Citric acid | 0.1 |
| (7) | (D) Sodium citrate | 0.9 |
| (8) | (E) Dimethylacrylamide/sodium acryloyldimethyltaurate crosspolymer | 0.2 |
| (9) | (F) Sorbitan tristearate | 0.1 |
| (10) | (F) Self-emulsifying glyceryl monostearate | 2 |
| (11) | (G) Glycerin | 10 |
| (12) | (G) 1,3-Butylene glycol | 5 |
| (13) | (G) Erythritol | 1 |
| (14) | (G) Cetanol | 5 |
| (15) | (G) Petrolatum | 3 |
| (16) | (G) Dimethicone | 5 |
| (17) | (G) Microcrystalline wax | 2 |
| (18) | (G) Meadowfoam oil | 5 |
| (19) | (G) Chelating agent | q.s. |
| (20) | (G) Antiseptic agent | q.s. |
| (21) | (G) Ion-exchanged water | Balance |
| Total | | 100 |
| Component (B)/(Component (A)+Component (F)) | | 0.056 |

### Formulation Example 4: Body cream (Table 11)

**[Table 11]**

| | Components | Content (mass%) |
|---|---|---|
| (1) | (A) POE (20) behenyl ether | 1 |
| (2) | (B) Sodium methyl stearoyl taurate | 0.3 |
| (3) | (C) Stearyl alcohol | 1 |
| (4) | (C) Behenyl alcohol | 3 |
| (5) | (D) Carnosine | 3.5 |
| (6) | (E) Sodium acrylate/sodium acryloyldimethyltaurate copolymer | 0.5 |
| (7) | (F) Sorbitan monostearate | 0.1 |
| (8) | (G) Glycerin | 15 |
| (9) | (G) 1,3-Butylene glycol | 10 |
| (10) | (G) Dipropylene glycol | 5 |
| (11) | (G) Microcrystalline wax | 1 |
| (12) | (G) Hydrogenated palm oil | 2 |
| (13) | (G) Mineral oil | 3 |
| (14) | (G) Hydrogenated polydecene | 3 |
| (15) | (G) Dimethicone | 2 |
| (16) | (G) Pentaerythrityl tetraethylhexanoate | 10 |
| (17) | (G) Chelating agent | q.s. |
| (18) | (G) Antiseptic agent | q.s. |
| (19) | (G) Ion-exchanged water | Balance |
| Total | | 100 |
| Component (B)/(Component (A)+Component (F)) | | 0.27 |

### Formulation Example 5: Whitening cream (Table 12)

**[Table 12]**

| | Components | Content (mass%) |
|---|---|---|
| (1) | (A) POE (20) behenyl ether | 1 |
| (2) | (B) Sodium N-stearoyl-L-glutamate | 0.1 |
| (3) | (C) Stearyl alcohol | 1 |
| (4) | (C) Behenyl alcohol | 3 |
| (5) | (D) Potassium 4-methoxysalicylate | 1 |
| (6) | (D) Betaine | 3 |
| (7) | (E) Sodium acrylate/sodium acryloyldimethyltaurate copolymer | 0.5 |
| (8) | (F) Sorbitan tristearate | 0.1 |
| (9) | (G) Glycerin | 10 |
| (10) | (G) 1,3-Butylene glycol | 7 |
| (11) | (G) Dipropylene glycol | 3 |
| (12) | (G) Myristyl myristate | 2 |
| (13) | (G) Petrolatum | 2 |
| (14) | (G) Triisostearin | 3 |
| (15) | (G) Hydrogenated polydecene | 2 |
| (16) | (G) Dimethicone | 1 |
| (17) | (G) Pentaerythrityl tetraethylhexanoate | 15 |
| (18) | (G) Hydrogenated polyisobutene | 1 |
| (19) | (G) Chelating agent | q.s. |
| (20) | (G) Antiseptic agent | q.s. |
| (21) | (G) Ion-exchanged water | Balance |
| Total | | 100 |
| Component (B)/(Component (A)+Component (F)) | | 0.091 |

### Formulation Example 6: Sunblock skincare cream (Table 13)

**[Table 13]**

| | Components | Content (mass%) |
|---|---|---|
| (1) | (A) POE (20) behenyl ether | 1.5 |
| (2) | (B) Sodium methyl stearoyl taurate | 0.3 |
| (3) | (C) Stearyl alcohol | 1 |
| (4) | (C) Behenyl alcohol | 3 |
| (5) | (D) L-ascorbic acid 2-glucoside | 2 |
| (6) | (E) Sodium acrylate/sodium acryloyldimethyltaurate copolymer | 1 |
| (7) | (F) Sorbitan tristearate | 0.2 |
| (8) | (G) Glycerin | 10 |
| (9) | (G) 1,3-Butylene glycol | 10 |
| (10) | (G) Dipropylene glycol | 5 |
| (11) | (G)Xylitol | 1 |
| (12) | (G) Hydrogenated polydecene | 10 |
| (13) | (G) Polybutylene glycol/PPG-9/1 copolymer | 2 |
| (14) | (G) Petrolatum | 2 |
| (15) | (G) Hydrogenated palm oil | 2 |
| (16) | (G) Dimethicone | 3 |
| (17) | (G) Octocrylene | 5 |
| (18) | (G) Phenylbenzimidazole sulfonic acid | 2.0 |
| (19) | (G) Allantoin | 0.1 |
| (20) | (G) Triethanolamine | q.s. |
| (21) | (G) Chelating agent | q.s. |
| (22) | (G) Antiseptic agent | q.s. |
| (23) | (G) Ion-exchanged water | Balance |
| Total | | 100 |
| Component (B)/(Component (A)+Component (F)) | | 0.18 |

### Formulation Example 7: Foundation cream (Table 14)

**[Table 14]**

| | Components | Content (mass%) |
|---|---|---|
| (1) | (A) Polyethylene glycol monostearate (40E.O.) | 1.5 |
| (2) | (B) Potassium cetyl phosphate | 0.2 |
| (3) | (C) Stearyl alcohol | 0.6 |
| (4) | (C) Behenyl alcohol | 0.8 |
| (5) | (D) Kojic acid | 1 |
| (6) | (D) Citric acid | 0.1 |
| (7) | (D) Sodium citrate | 0.9 |
| (8) | (E) Dimethylacrylamide/sodium acryloyldimethyltaurate crosspolymer | 0.2 |
| (9) | (F) Sorbitan tristearate | 0.1 |
| (10) | (F) Self-emulsifying glyceryl monostearate | 2 |
| (11) | (G) Glycerin | 10 |
| (12) | (G) 1,3-Butylene glycol | 5 |
| (13) | (G) Erythritol | 1 |
| (14) | (G) Cetanol | 5 |
| (15) | (G) Petrolatum | 3 |
| (16) | (G) Dimethicone | 5 |
| (17) | (G) Microcrystalline wax | 2 |
| (18) | (G) Meadowfoam oil | 5 |
| (19) | (G) Yellow iron oxide | q.s. |
| (20) | (G) Red iron oxide | q.s. |
| (21) | (G) Chelating agent | q.s. |
| (22) | (G) Antiseptic agent | q.s. |
| (23) | (G) Ion-exchanged water | Balance |
| Total | | 100 |
| Component (B)/(Component (A)+Component (F)) | | 0.056 |

The oil-in-water emulsion composition of the present invention has been described according to the foregoing embodiments and examples, but the invention is not limited to the foregoing embodiments and examples and may encompass various transformations, modifications, and improvements made to the various disclosed elements (including elements disclosed in the Claims, Description, and Drawings) within the scope of the invention and according to the fundamental technical idea of the present invention. Further, various combinations, substitutions, and selections of the various disclosed elements are possible within the scope of the claims of the invention.

Further issues, objectives, and embodiments (including modifications) of the present invention are revealed also from the entire disclosure of the invention including the Claims.

The numerical ranges disclosed herein are to be construed in such a manner that arbitrary numerical values and ranges falling within the disclosed ranges are treated as being concretely described herein, even where not specifically stated.

### Industrial Applicability

The composition of the present disclosure can suitably be used for external preparations for skin (external skincare preparations) and/or cosmetics to be applied to the skin.

## Claims

1. An oil-in-water emulsion composition comprising:
component (A): from 0.1 to 5% by mass of a hydrophilic nonionic surfactant;
component (B): more than 0.02% by mass to less than 0.5% by mass of an anionic surfactant;
component (C): from 0.5 to 6% by mass of a higher alcohol; and
component (D): from 2 to 30 mmol/100 g of an organic acid and/or a salt and/or derivative thereof, wherein
the component (A) includes a polyoxyalkylene alkyl ether and/or a polyalkylene glycol fatty acid ester.

2. The composition according to claim 1, wherein the component (A) has an HLB of 9 or greater.

3. The composition according to claim 1 or 2, wherein the component (B) includes at least one compound selected from the group consisting of N-acyl methyl taurates, N-acyl glutamates, and phosphate ester salts.

4. The composition according to any one of claims 1 to 3, wherein the component (C) includes a C₁₄₋₂₂ linear saturated alkyl group.

5. The composition according to any one of claims 1 to 4, wherein the component (D) includes citric acid, 4-methoxysalicylic acid, L-ascorbic acid, kojic acid, and/or 1-piperidinepropionic acid, and/or a salt and/or derivative thereof.

6. The composition according to any one of claims 1 to 5, further comprising:
component (E): from 0.2 to 1% by mass of a thickener.

7. The composition according to claim 6, wherein the component (E) includes a homopolymer and/or copolymer of 2-acrylamido-2-methylsulfonic acid or a salt thereof.

8. The composition according to any one of claims 1 to 7, further comprising:
component (F): from 0.1 to 2% by mass of a lipophilic nonionic surfactant.

9. The composition according to claim 8, wherein the lipophilic nonionic surfactant has an HLB of from 2 to 5.

10. The composition according to claim 8 or 9, wherein the component (F) includes a sorbitan fatty acid ester and/or a glycerin fatty acid ester.

11. The composition according to any one of claims 8 to 10, wherein a value found by dividing the mass of the component (B) by the total mass of the component (A) and the component (F) is from 0.003 to 0.3.

12. The composition according to any one of claims 1 to 11, wherein:
the component (A) includes a polyoxyalkylene alkyl ether; and
the average number of moles of polyoxyalkylene groups added in the polyoxyalkylene alkyl ether is from 10 to 40.

13. The composition according to any one of claims 1 to 11, wherein:
the component (A) includes a polyalkylene glycol fatty acid ester; and
the average number of moles of polyalkylene glycol groups added in the polyalkylene glycol fatty acid ester is from 25 to 55.

14. The composition according to any one of claims 1 to 13, wherein an average particle size of emulsion particles is less than 5 µm.

15. The composition according to any one of claims 1 to 14, wherein the composition has a hardness of from 8 to 22.
